Europäisches Patentamt

European Patent Office

Office européen des brevets

(19)

(11) Publication number: **0 112 064**
**B1**

(12) **EUROPEAN PATENT SPECIFICATION**

(45) Date of publication of patent specification: **13.08.86**

(21) Application number: **83307063.4**

(22) Date of filing: **18.11.83**

(51) Int. Cl.⁴: **B 01 J 21/12, C 07 C 1/04,**
**C 07 C 1/20**

(54) Improved catalysts.

(30) Priority: **10.12.82 GB 8235330**
**11.02.83 GB 8303772**

(43) Date of publication of application:
**27.06.84 Bulletin 84/26**

(45) Publication of the grant of the patent:
**13.08.86 Bulletin 86/33**

(84) Designated Contracting States:
**BE DE FR NL SE**

(56) References cited:
**EP-A-0 039 996**
**EP-A-0 068 603**
**FR-A-2 268 771**
**FR-A-2 418 024**
**US-A-4 009 199**
**US-A-4 350 772**

(73) Proprietor: **Coal Industry (Patents) Limited**
**Hobart House Grosvenor Place**
**London SW1X 7AE (GB)**

(72) Inventor: **Robinson, Joseph Gordon**
**Claybrook The Hyde**
**Winchcombe Gloucestershire (GB)**
Inventor: **Barnes, David Ian**
**70 Fiddlers Green Lane**
**Cheltenham Gloucestershire (GB)**
Inventor: **Riemer, Pierce William Foster**
**27 Lambourne Avenue**
**Huntly Gloucestershire (GB)**

(74) Representative: **Wood, John Irwin**
**Hobart House Grosvenor Place**
**London SW1X 7AE (GB)**

Courier Press, Leamington Spa, England.

**Description**

This invention concerns improved catalysts, more particularly it concerns improved catalysts of a synthetic zeolite type.

Synthetic zeolite catalysts, such as those produced by Mobil under the designation ZSM-5, are known to convert methanol, synthesis gas and many oxygen-containing organic compounds into saturated and unsaturated lower hydrocarbons, offering the possibility of gasoline production. In our copending British Application No. 82/11371, GB—A—2,100,710 (published 6.1.83), we have described a novel synthetic zeolite catalyst which comprises a highly porous amorphous silica xerogel having a layer of an amphoteric metal compound chemically bonded onto up to 90% of the available surface, the catalyst having a maximum pore diameter of about 1.5 nm. The metal is preferably aluminium, thereby giving a synthetic catalyst having an active aluminosilicate layer which may exist in one or two major crystal planes. It is now thought that the aluminosilicate layer has free aluminium-derived cations associated therewith and that cationic species such as $Al(OH)_2+$ may balance negatively charged 4- co-ordinate aluminium bonded to the surface of the silica xerogel. This and other explanations contained herein are to be regarded as theoretical only and have no bearing on the scope of the present invention.

It has been proposed in US Patent No. 4,009,199 to produce catalysts especially suitable for the esterification of dimethyl terephthalate by impregnating silica gel with a solution of an aluminium, titanium, zinc or tin compound, separating the excess solution and drying at elevated temperature to remove the solvent. Preferably, the catalyst is treated with a methanol/steam mixture at 150°C before use.

The present invention provides a process for the production of synthetic catalysts comprising the steps of:

a) treating a dry highly porous amorphous silica xerogel with anhydrous hydrolysable compound of aluminium in an amount sufficient to cover up to 90% of the BET surface area of the silica with a mono-layer of the compound and removing the solvent;

b) causing the surface of the silica to react with the compound by heating to a temperature of from 300°C to 1200°C, to yield a material having a maximum pore diameter of about 1.1 nm; and

c) treating the product material of step b) with an ammonium salt solution capable of exchanging labile cations for ammonium ions, washing the treated material with deionised water until no further ammonium cations can be detected, optionally ion exchanging to deposit transition metal cations on the surface, drying and calcining the material at a temperature not exceeding 1200°C to yield the catalyst.

The invention also provides a synthetic catalyst which comprises a highly porous amorphous silica xerogel having a layer of an aluminium compound chemically bonded onto the silica surface in an amount equivalent to a monolayer on up to 90% of the BET surface area of the silica, the catalyst having a maximum pore diameter of about 1.1 nm and having substantially only protons and/or transition metal cations as electrical charge balancing species on the surface of the layer, the reaction causing bonding of the layer having been carried out at a temperature of from 300 to 1200°C and the catalyst having been calcined at a temperature not exceeding 1200°C.

Steps a) and b) are preferably carried out by a method derived from our British Published Application GB—A—2,100,710, by treating the highly porous amorphous silica xerogel, having a suitable porosity, with a solution of the predetermined quantity of the hydrolysable compound of aluminium, removing the solvent to leave a layer of the compound on up to 90% of the available surface area, and causing the surface of the silica to react with the layer to chemically bond the aluminium to the surface of the silica. We have found that the temperature required in the reaction step b) is suitably 400 to 900°C, and that variations in this temperature can alter the catalytic properties of the final product. Preferably the reaction temperature is 450 to 550°C, especially about 500°C. Preferred catalysts have a deposition of aluminium compound corresponding to about 50% coverage.

Preferably, the silica xerogel having the deposited layer is treated in step c) with a dilute aqueous solution of an ammonium salt such as ammonium nitrate. The material is conveniently treated packed in a column, through which the solution may be passed until analysis demonstrates that no further aluminium cations are found in the eluent. The solution is then displaced by deionised water, which is passed until no further trace of ammonium ion is detected; this is conveniently done by comparing the pH of the eluent with that of the deionised water feed. These treatments are conveniently done under ambient conditions of temperature and pressure, but may also be carried out below or above ambient.

An ion-exchange step may be incorporated. Such ion-exchange steps have been described in the literature and can be carried out in manner known *per se*, suitably using a salt such as a nitrate. Preferred metals are lanthanum and rhodium.

After washing, the catalyst material is dried, conveniently under reduced pressure, and then calcined, preferably at a temperature of 300—900°C, more preferably 450—600°C, and suitably for 4 to 10 hours.

The catalyst of the invention is especially useful in the conversion of oxygen-containing aliphatics to hydrocarbons and water, such organics being preferably selected from alcohols and ethers and especially methanol. The catalyst may also be used, alone or in combination with, that is admixed with or directly subsequent to, a second catalyst, for the direct conversion of synthesis gas. Such a second catalyst may be a Fischer-Tropsch type catalyst such as a transition metal, for example iron, catalyst. Alternatively, the synthesis gas may be converted to methanol using a catalyst carrier impregnated with zinc and chromium

2

or zinc and copper, or impregnated with a lanthanide metal such as ruthenium. For the conversion of methanol, the catalyst may be used in a conventional reactor, using fixed, moving or fluidised beds. Temperatures are preferably in the range 300 to 500°C, especially 350 to 450°C. The Liquid Hourly Space Velocity (LHSV) is preferably from 0.1 to 1 $h^{-1}$, more preferably 0.2 to 0.5 $h^{-1}$. Under preferred conditions, the catalyst gives high conversion yields, approaching 100% per pass, and demonstrates an unusual and very high selectivity towards aromatics. This selectivity is materially and unexpectedly different from that observed using the untreated starting material catalyst, from that using the Na-form of the catalyst and from that found using Mobil's ZSM-5 catalyst.

Thus, the invention also provides a process for the conversion of an oxygen-containing aliphatic compound into hydrocarbons, comprising passing the compound in vapour form, over the catalyst at a temperature of 250 to 500°C and at a LHSV of 0.1 to 1 $h^{-1}$.

The optimum reaction temperature of step b) and the calcination temperature of step c) are thought to be about 500°C and 550°C, respectively, for a catalyst for the conversion of oxygen-containing aliphatic compounds into hydrocarbons.

We have also discovered a particularly unexpected activity of the catalysts of the invention. It is suggested in British Patent Specification No. 1,589,857 that crystalline zeolite catalysts catalyse the conversion of methanol with added water with selectivity to ethylene and propylene. This was not observed using the amorphous aluminosilicate catalysts of this invention, which yield a range of $C_1$—$C_7$ alkylbenzenes, but certain catalysts exhibited a very high selectivity to toluene.

Accordingly, the present invention further provides a process for the production of toluene with high selectivity, comprising passing methanol with at least an equal weight of water over a catalyst according to the invention which has been calcined at a temperature not exceeding 600°C, preferably not exceeding 450°C, said catalysts having substantially only protons as electrical charge balancing species on the surface of the layer, at a reaction temperature of 280 to 600°C, preferably 300 to 450°C.

The process reaction may be carried out using known forms of catalytic reactors, especially fluidised or fixed bed reactors. Suitable liquid hourly space velocities are in the range 0.1 to 5.0 $h^{-1}$, preferably 0.2 to about 1 $h^{-1}$. The reaction is conveniently carried out at atmospheric pressure, but pressures above or below atmospheric may be used.

The temperature of calcination of the catalysts which exhibit the high selectivity to toluene appears to be important, and is preferably less than 450°C, more preferably 300 to 350°C, although the other catalysts according to the invention are useful in the synthesis of a broader group of cyclic-hydrocarbons.

The concentration of the water/methanol feedstock is preferably in the region 3:1 to 4:1 by weight.

Under preferred conditions, the selectivity for toluene is in excess of 90%, with an overall conversion of methanol in the 30 to 50% range.

The preparation of catalysts according to the invention and their use in catalytic processes will now be described by way of example only.

Example 1

A sample (100 g) of commercial silica xerogel was heated in an oven for 4 hours at 120°C to remove physically absorbed water. The dried xerogel was cooled in a desiccator and immersed in a 250 ml of a 33.87% w/v solution of aluminium sec-butoxide in dry hexane (an amount sufficient to cover 50% of the BET surface with a monolayer of the aluminium alkoxide after removal of the solvent) and the pressure reduced to allow liquid to enter the pores of the xerogel. After 12 hours, excess solution was decanted and the material transferred to a vacuum oven where it was dried. After soaking in deionised water for 16 hours to complete hydrolysis of the alkoxide and decanting the liquor, the material was dried in the vacuum oven. The material was heated at 500°C for 4 hours to complete reaction of the aluminium compound with the surface of the xerogel. The product was designated "Catalyst A".

A sample of Catalyst A was packed into a column and a 0.1M aqueous solution of ammonium nitrate was pumped through the column. Samples of the eluent were tested periodically for the presence of aluminium by the addition of a few drops of 0.01M sodium hydroxide solution; samples containing aluminium cation were characterised by the formation of a gelatinous precipitate. When no further aluminium could be detected in this way, the ammonium nitrate solution was replaced by deionised water and pumping continued until the pH of the eluent was identical with that of the feed water. The product was removed from the column, dried under reduced pressure and thereafter calcined at 500°C for 6 hours. This was designated "Catalyst B".

High resolution electron microscopy of Catalyst B showed that it was composed of particles of 2—10 nm diameter having a strongly electron scattering surface layer which was an integral part of the silica matrix. There was little fine microporosity buh the gaps between the particles were approximately 1.0 nm.

Degassed methanol was pumped by a positive displacement pump through a preheater packed with glass beads before the methanol vapour was passed into a tubular reactor packed with the catalyst on test. The tubular reactor was run at various temperatures, 300, 350, 400 and 450°C, and the preheater was maintained at 5°C below the reaction temperature. The products emerging from the reaction tube were collected as a liquid fraction in an ice-cooled trap and a gaseous fraction in a gas reservoir. The products were analysed and the results are shown in Table 1 below.

3

It will be observed from the results that using Catalyst B, according to the invention, especially at a LHSV of 0.3 hr⁻¹ a very high specificity for aromatics is demonstrated.

Similar tests were performed using a form of Catalyst B that had been calcined at 325°C, and a conversion of methanol to hydrocarbons of 19.1% was obtained at a bed temperature of 350°C. A form of Catalyst B, calcined at 700°C, converted methanol to hydrocarbons in a yield of 69% at a bed temperature of 350°C, with high selectivity to aromatics.

The Na-form of Catalyst A, that is Catalyst A ion-exchanged with sodium was tested in the same apparatus and at a bed temperature of 450°C at a LHSV of 1.0 h⁻¹. A conversion of less than 3% was observed; no aromatic hydrocarbons were detected in the products.

## TABLE 1

| Catalyst | ZSM-5[a] | Catalyst A | | Catalyst B | | | |
|---|---|---|---|---|---|---|---|
| LHSV (h⁻¹) | 1.0 | 0.3 | 0.3 | 0.3 | 0.3 | 1.0 | 1.0 |
| Temperature (°C) | 370 | 350 | 450 | 350 | 450 | 350 | 450 |
| Conversion (wt.%) | 100 | 85 | 95 | 99.8 | 98.7 | 39.0 | 64.4 |
| Product distribution (wt.%) | | | | | | | |
| Methanol | 1.0 | 2.40 | 11.20 | 0.03 | 7.70 | 3.70 | 8.60 |
| Ethane | 0.6 | 9.10 | 16.90 | 0.50 | 3.20 | 0.20 | 0.90 |
| Propane | 16.2 | 6.20 | 7.20 | 0.01 | 1.30 | 0.10 | 1.80 |
| Butanes | 24.3 | 2.80 | 9.40 | 1.05 | 0.02 | 0.05 | 1.90 |
| Pentanes | 9.1 | 1.10 | 3.00 | 0.02 | 0.02 | — | 0.10 |
| C5⁺ Alkanes | 4.3 | 4.40 | 7.50 | 0.12 | 0.03 | 0.10 | 0.02 |
| Ethane | 0.5 | 7.60 | 10.40 | 0.01 | 5.00 | 0.10 | 1.20 |
| Propene | 1.0 | 3.90 | 4.30 | 0.40 | 0.20 | — | 0.10 |
| But-1-ene | 1.3 | — | — | 0.20 | 0.10 | — | — |
| Hexene | — | — | — | 0.02 | — | — | 0.10 |
| Benzene | 1.7 | — | — | — | — | — | — |
| C₇ Alkylbenzene | 10.5 | 5.80 | 7.00 | 7.20 | 3.70 | 2.90 | 2.70 |
| C₈ '' | 18.0 | 8.90 | 5.10 | 5.40 | 4.70 | 1.10 | 2.70 |
| C₉ '' | 7.5 | 3.20 | 5.90 | 2.30 | 8.60 | 1.30 | 2.00 |
| C₁₀ '' | 3.3 | 10.80 | 1.50 | 31.30 | 27.60 | 2.10 | 3.80 |
| C₁₁ '' | 0.2 | 9.70 | 0.60 | 9.00 | 7.10 | 0.80 | 1.80 |
| C₁₂ '' | — | 9.90 | 0.60 | 15.00 | 6.40 | 1.50 | 0.80 |
| C₁₃ '' | — | 13.90 | 8.90 | 25.40 | 20.10 | 2.90 | 2.00 |
| % Aromatics | 41.5 | 62.9 | 28.6 | 97.6 | 81.4 | 73 | 52 |

Note: [a]Results published in J. Catal. 1977, *47*. 249.

Degassed methanol-water mixtures were pumped by a positive displacement pump through a preheater packed with glass beads before the methanol/water vapour was passed into the tubular reactor packed with the catalyst, at a liquid hourly space velocity of $1.0 \, h^{-1}$. The tubular reactor was run at various temperatures, 250, 300, 350, 400 and 450°C, and the preheater was maintained at 5°C below the reaction temperature. The products emerging from the reaction tube were collected as a liquid fraction in an ice-cooled trap and a very small gaseous fraction (insufficient for analysis) in a gas reservoir. The products were analysed and the results are shown in Table 2 below.

TABLE 2

Water/Methanol wt. ratio 3:1

| Bed temperature (°C) | 250 | 300 | 350 | 400 | 450 |
|---|---|---|---|---|---|
| Methanol conversion (wt %) | 3.2 | 47 | 49 | 41 | 27 |
| Cycloalkanes (wt. %) | — | 1.1 | 0.8 | 1.2 | 3.1 |
| Toluene (wt %) | — | 93.5 | 95.7 | 92.5 | 90.0 |
| $C_2$ alkyl benzene (wt. %) | — | 3.9 | 2.5 | 4.3 | 6.0 |
| $C_3$ alkyl benzene (wt. %) | — | 1.5 | 0.9 | 1.9 | 0.9 |

The above-described procedure was repeated, except that the catalyst was calcined at 600°C for 4 hours, and the results are shown in Table 3 below.

TABLE 3

Water/Methanol wt. ratio 3:1

| Bed temperature (°C) | 250 | 300 | 350 | 400 | 450 |
|---|---|---|---|---|---|
| Methanol conversion (wt. %) | 0 | 22 | 32 | 48.1 | 50.2 |
| Cycloalkanes (wt. %) | — | 4.7 | 1.5 | 2.6 | 4.6 |
| Toluene (wt. %) | — | 42.1 | 41.8 | 32.0 | 15.8 |
| $C_2$ alkyl benzene (wt. %) | — | 22.1 | 10.4 | 2.2 | 3.0 |
| $C_3$ alkyl benzene (wt. %) | — | 10.1 | 7.8 | 6.1 | 5.2 |
| $C_4$ alkyl benzene (wt. %) | — | 7.7 | 10.0 | 17.2 | 19.1 |
| $C_5$ alkyl benzene (wt. %) | — | 10.5 | 11.1 | 18.9 | 28.2 |
| $C_6$ alkyl benzene (wt. %) | — | 1.2 | 15.3 | 19.8 | 21.1 |
| $C_7$ alkyl benzene (wt. %) | — | 1.2 | 2.0 | 1.1 | 3.0 |

It can be seen that although toluene is still a major product, the selectivity has been substantially reduced. The spread of products is broadly similar to that when pure methanol is used as feedstock under the same conditions, instead of water/methanol.

Instead of 3:1 water/methanol, a 1:2 water/methanol mixture was used, using the catalyst calcined at 600°C. The results are shown in Table 4 below, from which it can be seen that there is no selectivity for toluene.

TABLE 4

Water/Methanol wt. ratio 1:2

| | 200 | 250 | 300 | 350 | 400 | 450 |
|---|---|---|---|---|---|---|
| Bed temperature (°C) | 200 | 250 | 300 | 350 | 400 | 450 |
| Methanol conversion (wt. %) | 2.6 | 10.7 | 25.4 | 38.2 | 49.8 | 60.1 |
| Cycloalkanes (wt. %) | 4.0 | 1.0 | 0.3 | 1.6 | 2.6 | 3.9 |
| Toluene (wt. %) | 9.5 | 11.1 | 11.0 | 8.2 | 6.9 | 9.9 |
| $C_2$ alkyl benzene (wt. %) | 16.5 | 16.2 | 14.8 | 13.7 | 10.8 | 12.0 |
| $C_3$ alkyl benzene (wt. %) | 19.7 | 20.9 | 19.7 | 17.2 | 16.1 | 14.8 |
| $C_4$ alkyl benzene (wt. %) | 22.0 | 19.7 | 18.1 | 17.0 | 17.0 | 16.9 |
| $C_5$ alkyl benzene (wt. %) | 21.0 | 21.6 | 19.8 | 23.2 | 26.8 | 22.1 |
| $C_6$ alkyl benzene (wt. %) | 4.9 | 8.2 | 15.2 | 17.9 | 19.7 | 18.2 |
| $C_7$ alkyl benzene (wt. %) | 2.4 | 1.3 | 1.1 | 1.2 | 1.2 | 2.3 |

Further tests using a pure methanol feedstock and using the catalyst calcined at 600°C showed a range of alkyl benzenes produced.

**Claims**

1. A process for the production of synthetic catalysts, characterised in that it includes the steps of:
a) treating a dry highly porous amorphous silica xerogel with an anhydrous hydrolysable compound of aluminium in an amount sufficient to cover up to 90% of the BET surface area of the silica with a monolayer of the compound and removing the solvent;
b) causing the surface of the silica to react with the compound by heating to a temperature of from 300°C to 1200°C to yield a material having a maximum pore diameter of about 1.1 nm, and
c) treating the product material of step b) with an ammonium salt solution capable of exchanging labile cations for ammonium ions, washing the treated material with deionised water until no further ammonium cations can be detected, optionally ion exchanging to deposit transition metal cations on the surface, drying and calcining the material at a temperature not exceeding 1200°C.

2. A process according to claim 1, characterised in that the amount of aluminium compound in step a) is sufficient to cover approximately 50% of the BET surface area of the silica.

3. A process according to claim 1 or 2, characterised in that the reaction temperature of step b) is from 400 to 900°C.

4. A process according to claim 3, characterised in that the reaction temperature is 450 to 550°C.

5. A process according to any one of the preceding claims, characterised in that the calcining temperature of step c) is from 300 to 900°C.

6. A process according to claim 5, characterised in that the calcining temperature is from 450 to 600°C.

7. A synthetic catalyst comprising a highly porous amorphous silica xerogel having a layer of an aluminium compound in an amount equivalent to a monolayer covering up to 90% of the BET surface area of the silica, chemically bonded onto the surface thereof, said catalyst being characterised by a maximum pore diameter of about 1.1 nm and having substantially only protons and/or transition metal cations as electrical charge balancing species on the surface of the layer, and further characterised in that the reaction causing bonding of the layer having been carried out at a temperature from 300 to 1200°C and the catalyst having been calcined at a temperature not exceeding 1200°C.

8. A catalyst according to claim 7, characterised in that the layer of aluminium compound is equivalent to a monolayer on about 50% of the BET surface area of the silica.

9. A catalyst according to claim 7 or 8, characterised in that the reaction temperature was 400 to 900°C.

10. A catalyst according to claim 7, 8 or 9, characterised in that the calcination temperature was 300 to 900°C.

11. A process for the conversion of an oxygen-containing aliphatic compound into hydrocarbons, characterised in that the compound in vapour form is passed over a catalyst according to any one of claims 7 to 10, at a temperature of 250 to 500°C and at a LHSV of 0.1 to 1 $h^{-1}$.

12. A process for the production of toluene, characterised in that methanol is passed with at least an equal weight of water over a catalyst according to any one of claims 7 to 10 and further characterised in that the catalyst has substantially only protons as electrical charge balancing species on the surface of the layer and the catalyst has been calcined at a temperature not exceeding 600°C, at a reaction temperature of 280 to 600°C.

**Patentansprüche**

1. Verfahren zur Herstellung von synthetischen Katalysatoren, dadurch gekennzeichnet, daß es folgende Schritte umfaßt:

a) es wird ein trockenes hoch poröses amorphes Siliciumdioxidxerogel mit einer wasserfreien hydrolisierbaren Aluminiumverbindung in einer Menge umgesetzt, die ausreicht, um 90 % der BET-Oberfläche des Siliciumdioxids mit einer Monoschicht der Verbindung zu bedecken und das Lösungsmittel wird entfernt.

b) die Oberfläche des Siliciumdioxids wird mit der Verbindung umgesetzt, indem auf eine Temperatur von 300°C bis 1200°C erwärmt wird, um ein Material zu erhalten, welches einen maximalen Porendurchmesser von etwa 1,1 nm aufweist, und

c) das Produktmaterial des Schritts b) wird mit einer Ammoniumsalzlösung behandelt, die in der Lage ist, labile Kationen gegen Ammoniumionen auszutauschen, das behandelte Material wird mit deionisiertem Wasser gewaschen, bis keine weiteren Ammoniumkationen festgestellt werden können, gegebenenfalls einem Ionenaustausch unterworfen, um Übergangsmetallkationen auf der Oberfläche abzulagern, das Material wird getrocknet und calciniert bei einer Temperatur, die 1.200°C übersteigt.

2. Verfahren nach Anspruch 1, dadurch gekennzeichnet, daß die Menge der Aluminiumverbindung des Schritts a) ausreicht, um etwa 50 % der BET-Oberfläche des siliciumdioxids zu bedecken.

3. Verfahren nach Anspruch 1 oder 2, dadurch gekennzeichnet, daß die Reaktionstemperatur des Schritts b) von 400 bis 900°C beträgt.

4. Verfahren nach Anspruch 3, dadurch gekennzeichnet, daß die Reaktionstemperatur 450 bis 550°C beträgt.

5. Verfahren nach einem der vorstehenden Ansprüche, dadurch gekennzeichnet, daß die Calcinierungstemperatur des Schritts c) von 300 bis 900°C beträgt.

6. Verfahren nach Anspruch 5, dadurch gekennzeichnet, daß die Calcinierungstemperatur von 450 bis 600°C beträgt.

7. Synthetischer Katalysator, der ein hochporöses amorphes Siliciumdioxidxerogel umfaßt, welches eine Schicht einer Aluminiumverbindung in einer Menge aufweist, die einer Monoschicht entspricht, die bis zu 90 % der BET-Oberfläche des Siliciumdioxids bedeckt, welche chemisch an dessen Oberfläche gebunden ist, welcher Katalysator gekennzeichnet ist durch einen maximalen Porendurchmesser von etwa 1,1 nm und im wesentlichen lediglich Protonen und/oder Übergangsmetallkationen als Spezien zum Ausgleich der elektrischen Ladung an der Oberfläche der Schicht aufweist, und der weiter dadurch gekennzeichnet ist, daß die Reaktion, die die Bindung der Schicht hervorruft, bei einer Temperatur von 300 bis 1.200°C durchgeführt worden ist und der Katalysator bei einer Temperatur calciniert worden ist, die 1.200°C nicht übersteigt.

8. Katalysator nach Anspruch 7, dadurch gekennzeichnet, daß die Schicht der Aluminiumverbindung einer Monoschicht von etwa 50 % der BET-Oberfläche des Siliciumdioxids äquivalent ist.

9. Katalysator nach Anspruch 7 oder 8, dadurch gekennzeichnet, daß die Reaktionstemperatur 400 bis 900°C betrug.

10. Katalysator nach Anspruch 7, 8 oder 9, dadurch gekennzeichnet, daß die Calcinierungstemperatur 300 bis 900°C betrug.

11. Verfahren zur Umwandlung einer sauerstoffhaltigen aliphatischen Verbindung in Kohlenwasserstoffe, dadurch gekennzeichnet, daß die Verbindung in Dampfform über einen Katalysator nach einem der Ansprüche 7 bis 10 bei einer Temperatur von 250 bis 500°C und einer stündlichen Flüssigkeitsraumströmungsgeschwindigkeit von 0,1 bis 1 $h^{-1}$ geleitet wird.

12. Verfahren zur Herstellung von Toloul, dadurch gekennzeichnet, daß Methanol mit wenigstens der gleichen Gewichtsmenge Wasser über einen Katalysator nach einem der Ansprüche 7 bis 10 geleitet wird, und weiter dadurch gekennzeichnet, daß der Katalysator im wesentlichen lediglich Protonen als Spezien zum Ausgleich der elektrischen Ladung an der Oberfläche der Schicht aufweist und der Katalysator bei einer Temperatur calciniert worden ist, die 600°C nicht übersteigt, bei einer Reaktionstemperatur von 280 bis 600°C.

**Revendications**

1. Procédé pour la production de catalyseurs synthétiques, caractérisé en ce qu'il comprend les étapes consistant à:

a) traiter un xérogel de silice amorphe sèche et hautement poreuse par un composé anhydre hydrolysable d'aluminium, en une quantité suffisante pour recouvrir d'une monocouche du composé jusqu'à 90 % de la surface spécifique BET de la silice, et enlever le solvent;

b) provoquer la réaction de la surface de la silice avec le composé, par chauffage jusqu'à une température de 300°C à 1200°C, pour obtenir une matière ayant un diamètre maximal des pores d'environ 1,1 nm, et

c) traiter la matière produite à l'étape b) par une solution d'un sel d'ammonium capable de remplacer des cations labiles et d'introduire par échange des ions ammonium, laver à l'eau désionisée la matière ainsi traitée, jusqu'à ce qu'on ne puisse plus déceler de cations ammonium, éventuellement soumettre à un échange d'ions pour provoquer le dépôt de cations de métaux de transition à la surface sécher et calciner la matière à une température n'excédant pas 1200°C.

2. Procédé selon la revendication 1, caractérisé en ce que la quantité du composé d'aluminium est suffisante, dans l'étape a), pour recouvrir environ 50 % de la surface spécifique BET de la silice.

3. Procédé selon la revendication 1 ou 2, caractérisé en ce que la température de réaction se situe entre 400 et 900°C à l'étape b).

4. Procédé selon la revendication 3, caractérisé en ce que la température de réaction va ce 450 à 550°C.

5. Procédé selon l'une quelconque des revendications précédentes, caractérisé en ce que la température de calcination est à l'étape c) de 300 à 900°C.

6. Procédé selon la revendication 5, caractérisé en ce que la température de calcination de situe entre 450 et 600°C.

7. Catalyseur synthétique, comprenant un xérogel de silice amorphe hautement poreuse, ayant une couche d'un composé d'aluminium présente en une quantité équivalente à une monocouche recouvrant jusqu'à 90 % de la surface spécifique BET de la silice, chimiquement liée à la surface de cette silice, ledit catalyseur étant caractérisé par un diamètre maximal des pores d'environ 1,1 nm et n'ayant essentiellement que des protons et/ou des cations de métaux de transition comme espèces pour contrebalancer la charge électrique à la surface de la couche, et étant caractérisé en outre en ce que la réaction provoquant la liaison de la couche a été effectuée à une température de 300 à 1200°C et en ce que le catalyseur a été calciné à une température n'excédant pas 1200°C.

8. Catalyseur selon la revendication 7, caractérisé en ce que la couche du composé d'aluminium équivaut à une monocouche sur environ 50 % de la surface spécifique BET de la silice.

9. Catalyseur selon la revendication 7 ou 8, caractérisé en ce que la température de réaction se situe entre 400 et 900°C.

10. Catalyseur selon la revendication 7, 8 ou 9, caractérisé en ce que la température de calcination se situe entre 300 et 900°C.

11. Procédé pour transformer un composé aliphatique oxygéné en des hydrocarbures, caractérisé en ce qu'on fait passer le composé, sous forme de vapeur, sur un catalyseur selon l'une quelconque des revendications 7 à 10, à une température de 250 à 500°C et à une VVH de 0,1 à 1 h$^{-1}$.

12. Procédé pour la production de toluène, caractérisé en ce qu'on fait passer du méthanol avec un poids au moins égal d'eau sur un catalyseur selon l'une quelconque des revendications 7 à 10, et caractérisé en outre en ce que le catalyseur ne comporte essentiellement que des protons comme espèce contrebalançant la charge électrique à la surface de la couche et en ce que le catalyseur a été calciné à une température n'excédant pas 600°C, à une température de réaction de 280 à 600°C.